# EUROPEAN PATENT APPLICATION

(11) **EP 1 130 086 A1**
(43) Date of publication of application: **05.09.2001**
(21) Application number: 00103537.7
(22) Date of filing: 18.02.2000
(51) Int. Cl.: C12N 5/08, G01N 33/50, C12Q 1/68

(54) **Genetically engineered keratinocytes and toxicity assay using said keratinocytes**

(71) Applicant: Deutsches Krebsforschungszentrum Stiftung des öffentlichen Rechts, 69120 Heidelberg (DE); TPSc (Technologies du Promoteur de Stress Cellulaire), 75009 Paris (FR)
(72) Inventor: Fusenig, Norbert, 69118 Heidelberg (DE); Ibghei, David, c/o TPSc, 75009 Paris (FR); Arrigo, Patrick, c/o Université de Lyon, 69622 Villeurbanne Cédex (FR)
(74) Representative: Schüssler, Andrea, Dr.

(57) **Abstract**

Described is an immortalized human skin keratinocyte line stably transfected with a vector, wherein said vector comprises a promoter inducible by environmental hazards and a reporter gene in operative linkage thereto. Preferably, this keratinocyte line is the line HaCaT. Furthermore, in vitro toxicity assays making use of these immortalized human skin keratinocyte lines are described.

## Description

The present invention relates to an immortalized human skin keratinocyte line stably transfected with a vector, wherein said vector comprises a promoter inducible by environmental hazards and a reporter gene in operative linkage thereto. In a preferred embodiment this keratinocyte line is the line HaCaT. The present invention also relates to in vitro toxicity assays making use of these immortalized human skin keratinocyte lines.

Human skin is the tissue most exposed to environmental insults such as heat, ultraviolet irradation, chemicals etc.. The steadily increasing number of environmental hazards to which the human skin is exposed necessitates sensitive and relevant assay systems to monitor their damaging potency and to test for protective and therapeutic means to reduce or eliminate acute and chronic skin damage. The number of pollutants and environmental agents is huge and their chemical/physical nature very heterogeneous or undefined ranging from sunlight (UVR and IR) over metal compounds and industrial chemicals to pesticides, herbicides and biocides, not to forget detergents, cosmetic and skin care products. At present, data on skin irritation are required by the various regulatory authorities for the notification of new and existing chemicals, agro-chemicals, cosmetic and skin care ingredients and medical devices. They are usually tested on the shaved skin of albino rabbits following the method of Draize (1944) and the OECD guideline 404 (van den Sandt, 1999). However, these methods have been critisized for the low reproducibility, long-assay duration and high costs, Moreover, due to increasing public and legislative pressure to reduce the number of animals used in safety testing and the practical and ethical limitations of conducting in vivo human studies, there is increasing need for the development and evaluation of reliable in vitro methods.

In addition to ethical issues, there are problems with extrapolating results of animal tests to humans due to different anatomy, metabolism and sensitivity (Tupker et al., 1997). Several in vitro test systems have been developed to predict local toxicity and irritation on skin, but none met so far the expectations of a sensitive and predictive assay (Botham et al., 1995; van den Sandt, 1999), with two exceptions: The in vitro corrosivity assay, for determining irreversible tissue damage (Fentem et al., 1998), and the phototoxicity assay (Spielmann et al., 1998). It is interesting to note that the assays validated for skin corrosivity testing consisted of structured epidermis ("TER"-assay and "EPISKIN"), although both involved rather crude endpoints and were, thus, not suited as indicators for low-level cytotoxicity. In vivo studies have clearly demonstrated that exposure of the skin to irritants can induce direct cytotoxicity and cell death of keratinocytes as well as an inflammatory response in the underlying dermis depending on the strength and nature of the irritant. These different actions cannot be strictly separated, because they are interdependent in a complex signalling mechanism, by which keratinocytes transform non-specific harmful stimuli from the environment into endogeneous signals (cytokines and prostagandins) thereby activating local and systemic repair and defence mechanisms (van Ruissen et al., 1998). These molecular changes clearly preceed any morphological signs of inflammation and are, thus, sensitive and early indicators of skin irritation. Thus, relevant in vitro test models should reflect most closely the known reactions of human skin irritancy at the molecular, cellular, and tissue level. They should provide a structured epidermis with typical barrier function and metabolizing capacity as well as the major dermal cellular and matrix components. The model should possess mechanistically-based indicator systems with quantitative and easy-to-measure endpoints and respond to a wide spectrum of environmental agents causing both direct and indirect skin alterations. The model should as well be suited for the assessment of preventive, protective and therapeutic means to reduce or repair skin damage (Weber et al., 1997). Consequently, in vitro skin or epidermal equivalents have been developed consisting of human keratinocytes forming a differentiated multilayered epithelium on top of a collagenous matrix with or without dermal cells (Damour et al., 1998).

The in vitro systems used so far for determining the cell toxicity were mainly based on mouse fibroblast lines (e.g. 3T3). The toxic effect of a particular factor on the skin was analysed by determining membrane damages which result in the increased uptake of particular dyes or the release of cellular enzymes (LDH) as well as by determining damages of mitochondria leading to a decreased metabolism of an indicator dye (MTT). By using these indicators damages of the cell by directly acting chemicals as well as UVB irradiation can be quantitatively assayed. However, by the use of these test systems the toxicity of compounds showing only a medium or low effect can not be evaluated. The reasons for this failure are inter alia most probably the inadequate and late endpoint measurements ("MTT"-assay) registering only severe cell damage. Cell culture systems using rather crude cell viability endpoints are more sensitive, but of lower predictibility and lipid or solid-phase products can not be adequately tested. In addition, substances which show a cell damaging effect only after having being metabolized (e.g. hydrocarbons) can not be detected by using the above systems due to the lack of appropriate enzymes in these mouse cells. The same holds for chemicals which only act indirectly (e.g. via oxygen radicals, ROS) or long-wave UVA the toxic effect of which could so far only be analysed by using a costly assay for the detection of products oxydized by these radicals. Thus, in skin irritation testing, none of the above in vitro systems was validated so far, except for predicting corrosivity (Fentem et al., 1998; van den Sandt, 1999). Finally, so far a cellular in vitro toxicity model based on normal human cells has not been established, thus, an extrapolation of the results as regards the proposed effect on the human skin is hardly possible.

Thus, the technical problem underlying the present invention is to provide a cellular in vitro system which is based on human cells and which allows a sensitive, reliable, cost saving and quantitative routine analysis of any cell damaging factor.

The solution to the above technical problem has been achieved by providing the embodiments characterized in the claims.

A novel, mechanism-based highly sensitive and quantitative in vitro assay for identifying and charactering environmental hazards affecting human skin has been developed. This assay utilizes molecular pathways rapidly induced during cellular irritation as indicators of low-level toxic effects, i.e. the sensitive assessment of both immediate and delayed skin reactions regulated through different molecular pathways, necessitates the analysis of the involved cellular response mechanisms. Thus, whereas UV-B directly induces DNA damage in keratinocytes, UV-A acts mostly through the generation of reactive oxygen species (ROS) producing photo-oxidative damage on nucleic acids, lipids and proteins both in keratinocytes and dermal cells. In addition, visible light and IR radiation, inducing heat, appear to contribute to the chronic effects of solar radiation on the skin (Boukamp et al., 1999). Both UV-B and heat, but also heavy metals, arsenics and various pesticides, industrial residues and chemicals including pharmaceuticals but also low frequency electromagnetic waves induce a family of cellular proteins called heat shock proteins (HSP) which are universally produced under stress. This stress response is considered an early and sensitive indicator of toxic insults. Another essential mediator is NF_{κ}B, an inducible transcription factor involved in the regulation of many genes expressed in cellular stress response. It is activated by various stimuli, such as UVR, ROS and proinflammatory cytokines (TNFα, IL-1). Since UVR but also ozone, many chemicals, chromium and nickel compounds cause skin irritancy through the formation of ROS, NF_{κ}B is considered a very sensitive indicator. Similar to NF_{κ} B but with different spectrum of gene activation, AP-1 is another major transcription factor regulating many genes involved in inflammation and stress response in skin. PTX3 is a prototypic long pentraxin and belongs to a family of highly conserved acute phase proteins which is expressed in endothelial cells in response to IL-1 and TNFα. A rather high specificity of induction and its localization in endothelial cells renders this promoter a specific indicator of toxic vascular damage. Further indirect indicators of skin damage are members of the pro-inflammatory cascade, such as II-1 and IL-6, induced in dermal fibroblasts, and ICAM-1, upregulated in fibroblasts and endothelial cells. Another member of the stress protein family is HSP-27 activated not only by heat but also in embryonic differentiation and by drugs with a teratogenic potential. More interestingly, the HSP-27 promoter also contains an estrogen box and will, thus, be a sensitive indicator of hormone-like acting compounds such as the growing family of endocrine disruptors (Arrigo, 1998). These anthropogenic agro-chemicals and phytoestrogens disrupt endocrine functions during development resulting in teratogenic and neoplastic alterations in wildlife and humans. Although all of these indicators are physiologically expressed in keratinocytes as well as other cells, their gene products are produced at a too low level to be quantitatively measured as endpoints of cellular stress. In order to overcome these severe disadvantage, in the immortalized human keratinocytes of the present invention promoter constructs of genes centrally involved in cellular stress and repair reactions were coupled with reporter gene constructs generating secreted products. These products are measured, e.g. in the culture supernatant, representing a highly sensitive and quantitative readout system. The constructs are stably transfected into established human keratinocytes. The resulting transgenic cell lines are used as a sensitive and biologically relevant monitoring system for a broad spectrum of toxic compounds, e.g. as conventional monolayer assays. In contrast to the rather crude and unspecified toxicity assessed in in-vitro assays of the prior art, the assay of the present invention substantially improves the analytical methods for toxicity testing by using specific metabolic endpoints induced by harmful agents in the cell. It is much more sensitive than conventional cytotoxicity assays and provides both quantitative and dose-dependent results of a large variety of individual agents. Moreoever, it allows the detection of directly and indirectly caused damages. Finally, because of its broad-based analytical capacity, it is uniquely suited to assay for mixtures of pollutants, industrial waste products and toxic substances provided they can be solubilized or dispersed in the culture medium.

Thus, in one aspect, the present invention relates to an immortalized human keratinocyte line stably transfected with a vector, said vector comprising (a) a promoter inducible by environmental hazards; and (b) a reporter gene in operative linkage thereto.

As used herein, the term "immortalized human keratinocyte line" relates to any immortalized human keratinocyte line which can be transfected with vectors, allows gene expression and is useful in the assay of the invention. Examples of suitable human cell lines are skin and mucosal keratinocytes immortalized by transfection of the viral oncogenes of Simian virus 40 (SV40-LT gene), of human papilloma viruses type 16, 18, 33 (HPV-E6/E7 gene) or by transfection of the catalytical subunit of the enzyme telomerase. In a preferred embodiment, this cell line is the cell line HaCaT or a cell line having the functional characteristics of HaCaT. HaCaT is a spontaneously immortalized human skin keratinocyte line (Boukamp et al., 1988) which has stably preserved most of the functional characteristics to form a differentiated epidermis in transplants on nude mice (Breitkreutz et al., 1988) as well as in organotypic cocultures with normal fibroblasts (Schoop et al., 1999).

The construction of the vectors needed for obtaining the genetically engineered immortalized human keratinocyte line of the invention can be carried out according to conventional methods (cf. Sambrook et al., 1989, Molecular Cloning: A Laboratory Manual, 2^{nd} edition, Cold Spring Harbor Laboratory Press, NY, USA). Suitable starting vectors allowing amplification in prokaryotic and eukaryotic expression systems including the immortalized human keratinocyte lines described above are, e.g. the "pGL-3" basic-vector (Promega GmbH, Mannheim, Germany) or any other shuttle vector allowing amplification in procaryotes and expression in eucaryotes. These vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts fo amplifying said vectors include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells; and plant cells.

Introduction of the above described vectors into the host cell can be effected by well known methods, e.g. calcium phosphate transfection, DEAE-dextran mediated transfection, cationic lipid-mediated transfection, electroporation, transduction, infection, or other methods. Such methods are described in many standard laboratory manuals.

As used herein, the term " a promoter inducible by environmental hazards" relates to any promoter which is useful in the in vitro system of the present invention, i.e. any promoter which is inducible by factors which show cell toxicity, e.g. can lead to a damage of cells. Such promoters and suitable sources for such promoters are well known to the person skilled in the art. The expression vectors (inducible promoter/reporter gene) will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated. Particular promoters are selected according to the nature of the stress factor to be analysed. As promoter constructs to serve as sensitive indicators for a broad spectrum of environmental hazards from UVR over chemicals to pesticides and industrial waste products, those are selected which promise both broad sensitivity and high specificity to certain kinds of agents. The promoters can be selected according to the known biologic mechanisms of skin irritation, i.e. consisting of both direct alterations induced primarily in the first-line target cells, the keratinocytes, and indirect effects caused in fibroblasts and endothelial cells by mediators (e.g. cytokines, prostaglandins) released by injured keratinocytes (Nickoloff et al., 1994). For example, the promoter of HSP-70, the heat shock protein 70 (Dreano et al, 1988), can be used to serve as a broad-range stress indicator, sensitive to activation by many agents including heat, UVR, reactive oxygen species (ROS), metals, and chemicals. HSP-27 is another cellular stress protein activated by a spectrum of events including disturbances of cell differentiation and is also induced by teratogenic as well as hormone-like compounds (Buzin et al., 1984; Arrigo, 1998). Thus, the HSP-27 promoter will serve as a sensitive indicator for endocrine disrupters (e.g. xenoestrogens) (Hickay et al., 1986). NF_{κ}B is a transcription factor activated by various stimuli and will, thus, serve as indicator both for direct (UVR) as well as indirectly (ROS) caused cell irritation. The transcription factor AP-1, has a similar, although different activation spectrum (Saliou et al., 1999). Thus, NF_{κ}B or AP-1 sites containing promoters can be used for assaying the above stress factors (Kretz et al., 1996).

Thus, in a preferred embodiment of the genetically engineered immortalized human skin keratinocyte line of the present invention the environmental hazard which induces the promoter in operative linkage with the reporter gene is heat, ultraviolet irradiation (UVR), ozone or an organic or anorganic compound, e.g. a metal, pesticide, herbicide, biocide or a combustion product.

Accordingly, a further preferred embodiment of the genetically immortalized human skin keratinocyte line is a cell line wherein the vector carries a promoter which is the HSP-70- or HSP-27-promoter or a NF_{κ}B- or AP-1-site containing promoter.

As used herein, the term "reporter gene" relates to any gene the product of which can be used in order to determine the rate of expression directed by the inducible promoter. Such reporter genes and assays for determining the expression rate of the reporter gene as well as for appropriately linking the reporter gene to the promoter are well known to the person skilled in the art. One example of such a reporter gene is the gene encoding the human placental secreted alkaline phosphatase (SEAP). The SEAP reporter gene encodes a truncated form of the placental alkaline phosphatase enzyme which lacks the membrane anchoring domain, thereby allowing the protein to be efficiently secreted by transfected cells (Rinderle, C., 1998). Levels of SEAP activity detected in the culture medium are directly proportional to changes in intracellular concentrations of SEAP mRNA and protein. SEAP is extremely heat-stable and resistant to the inhibitor L-homoarginine, so that endogenous alkaline phosphatase activity can be eliminated by pretreatment of samples at 65°C and incubation with this inhibitor. A commercial detection kit composed of chemiluminescent substrate (CSPD) and the fluorescent substrate 4-methylumbelliferyl phosphate (MUP) enables to monitor expression of the SEAP reporter gene through simple, sensitive, non-radioactive assays (e.g. provided by Fa. Tropix/Becton-Dickinson, Heidelberg, Germany). The chemiluminescent assay can detect as little as 10⁻¹³ g of SEAP protein, making it one of the most sensitive enzymatic reporters available comparable to assays for firefly luciferase. The detection assay is linear over a 10⁴-fold range of enzyme concentrations, which makes it particularly well-suited for dose-response studies and comparative analyses. The secreted nature of SEAP provides several advantages for the use of this enzyme as a transcription reporter, because i) preparation of cell lysates is not required, ii) the kinetics of gene expression can be studied simply be repeated collection of the culture medium from the same cultures, iii) toxicity assays after both single and multiple exposure of cells to damaging agents can be performed simultaneously, iv) sample collection of the culture medium can be automated in 96-well plates, and iv) background from endogenous alkaline phosphatase is almost absent in the culture medium following pretreatment. Additional well known reporter molecules that can be used instead of SEAP, which are, however, not secreted into the culture medium include chloramphenicol acetyltransferase (CAT), luciferase, alkaline phosphatase, ß-galactosidase, green fluorescent protein (GFP) or any protein detectable by an chemiluminescent assay or an antibody.

Thus, in a further preferred embodiment of the immortalized human skin keratinocyte line of the present invention the reporter gene is the gene encoding luciferase, chloramphenicol acetyl transferase (CAT), green fluorescent protein (GFP) or human placental secreted alkaline phosphatase (SEAP).

Finally, the present invention also relates to an in vitro toxicity assay, comprising (a) culturing the immortalized human skin keratinocyte line of the present invention in the presence of the candidate environmental hazard under conditions such that the protein encoded by the reporter gene is expressed depending on induction of the promoter; and (b) determining whether expression of the reporter gene occurs, wherein expression of the reporter gene is indicative of cell toxicity of the candidate environmental hazard. This in vitro toxicity assay can also be carried out by (a) culturing the immortalized human skin keratinocyte line of the present invention in (i) the presence and (ii) absence of the candidate environmental hazard under conditions such that the protein encoded by the reporter gene is expressed depending on induction of the promoter; and (b) comparing the expression rate of the reproter gene of (i) and (ii) wherein an increase of the expression rate of (i) compared to (ii) is indicative of cell toxicity of the candidate environmental hazard. These assays, i.e. the culturing of the cell, contacting the cell with the candidate environmental hazard und the determination of the concentration of the protein expressed from the reporter gene can be carried according to well known routine methods and assays, e.g. the methods and assays described in the examples, below.

Under the Budapester Treaty the following culture has been deposited with the "Deutschen Sammlung von Mikroorganismen und Zellkulturen GmbH (DSMZ), Mascheroder Weg 1b, Braunschweig, Germany" on October 27, 1999:
HaCaT-HSP70/Cat DSM ACC2417

### Brief description of the Figures:

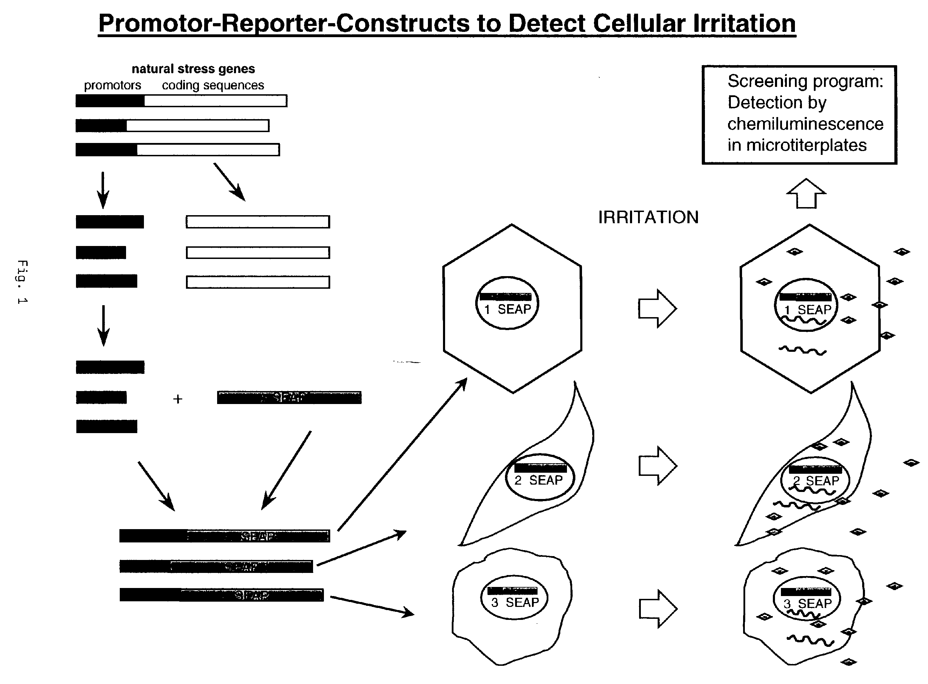

### Figure 1: Scheme of the in vitro toxicity assay

SEAP is given as an example of a reporter gene the expression of which is detectable via chemiluminescence measurement.

### Figure 2: Map of the plasmid Hsp70p17catneo

The human HSP-70 promotor present in the plasmid pl7catneo (Kretz and Arrigo, 1994) contains 600 bp of the HSP-70 gene (500 bp of the non-transcribed sequence and 113 bp of the RNA leader). For construction of the plasmid the 1.5 kbp XbaI-BamHI element containing the cat-gene (Dreano et al., 1988) was inserted in pl7hgHneo (6,7 kbp) (Dreano et al., 1988) after removing the hgH gene.

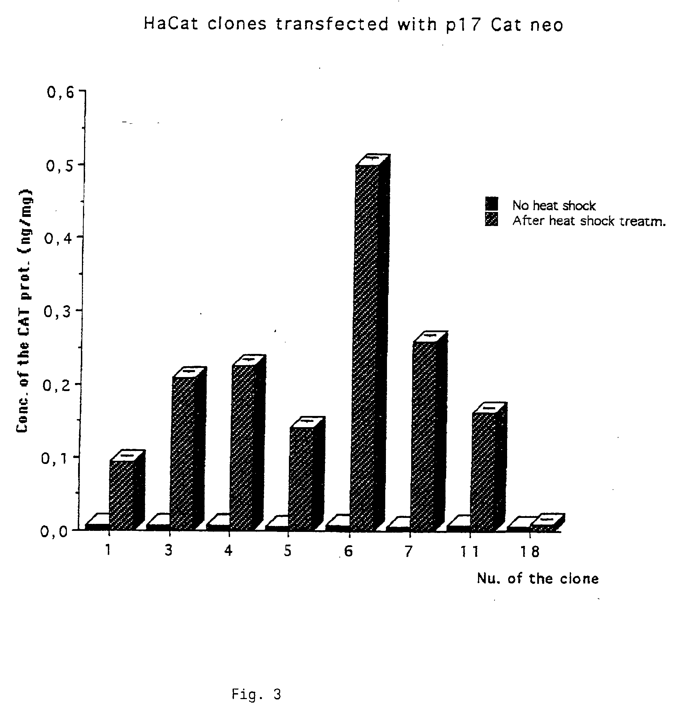

### Figure 3: Determination of the induction of the HSP-70 promoter in HaCaT cells by heat shock

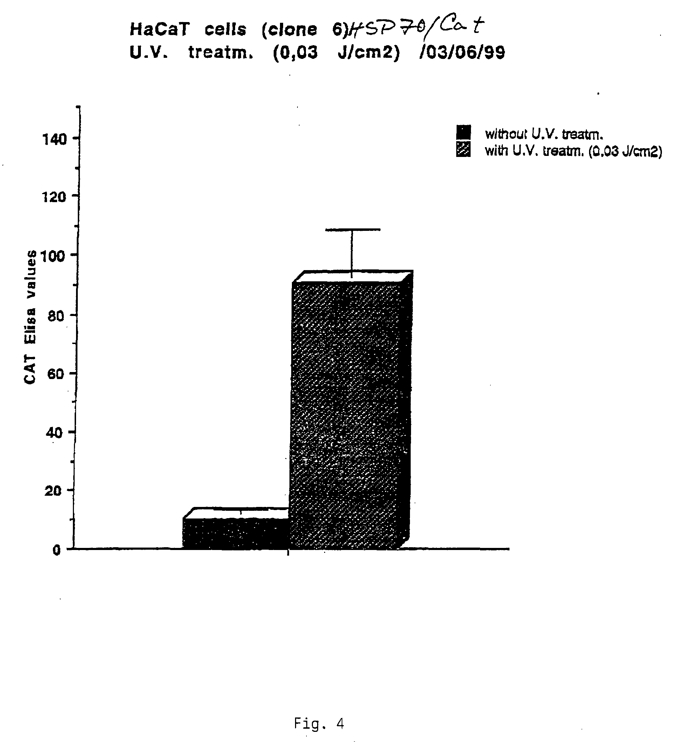

### Figure 4: Determination of the induction of the HSP-70 promoter in HaCaT cells by radiation with UVA

The following examples further explain the present invention.

### Example 1: Transfection of the vector Hsp70p17catneo in the HaCaT cells

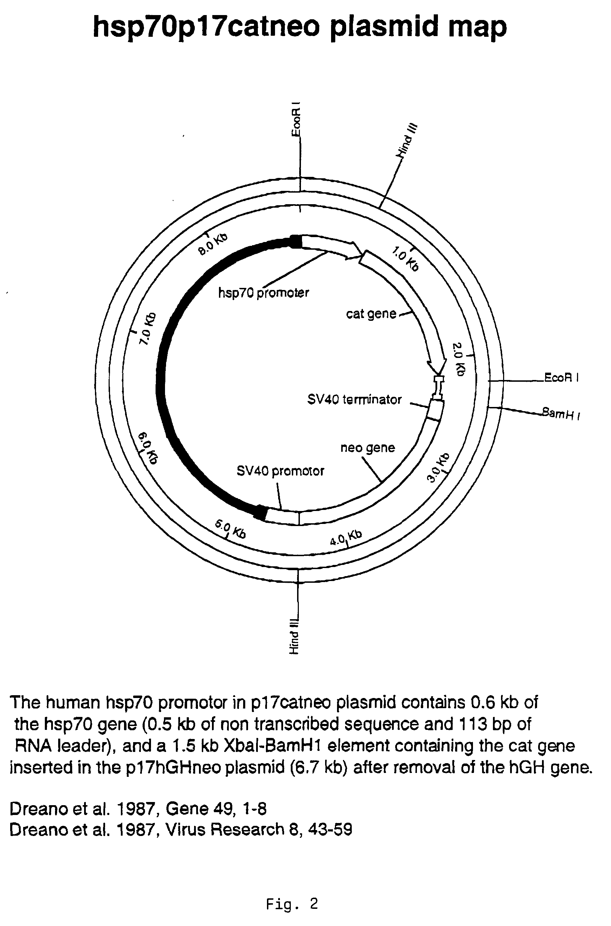

The vector as shown in Fig. 2 (recloning of the vector pl7catneo as described in Dreano et al., 1988) was transfected into HaCaT cells by lipofectamine procedure following the instructions of the manufacturer (Gibco-BRL, Eggenstein, 'Germany). The selection of transfected cells was carried out for one month by using Geniticin (G418) and surviving clones were expanded by standard culture technique.

### Example 2: Determination of the induction of the HSP-70 promoter in HaCaT cells by heat shock

8 clones (HaCaT cells transfected with the vector of Example 1) were exposed in an incubator for 90 min. to an elevated temperature of 43°C in culture medium (DMEM plus 5% fetal calf serum). This step was omitted when providing control cells. After further incubation at 37°C for 18 hrs for cell recovery and reporter gene expression, cells were lysed und 50 *µ*g of total protein were analysed with respect to expression of the cat-gene with an ELISA (Roche Diagnostics, Mannheim, Deutschland). As shown in Figure 3, the untreated control cells do not exhibit expression of the cat-gene. However, cells incubated at the elevated temperature show an increased expression of the cat-gene varying between different clones.

### Example 3: Determination of the induction of the HSP-70 promoter in HaCaT cells by radiation with UVA (395-400 nm)

HaCaT clone 6 was exposed to UVA radiation (0,03 J/cm²) by a thin film of culture medium in the Petri dish. This step was omitted when providing control cells. 50 *µ*g of total protein were analysed with respect to expression of the cat-gene with an ELISA (Roche Diagnostics, Mannheim, Deutschland). As shown in Figure 4, the untreated control cells do not exhibit expression of the cat-gene. However, cells irradiated with low dose UV show an increased expression of the cat-gene.

### List of References

Arrigo A-P. Small stress proteins: chaperones that act as regulators of intracellular redox state and programmed cell death. Biol. Chem. 379:19-16 (1998)
Botham PA, Chamberlain M, Barratt MD, Curren RD, Esdaile DJ, Gardner JR, Gordon VC, Hildebrand B, Lewis RW, Liebsch M, Logemann P, Osborne R, Ponec M, Regnier J-F, Steiling W, Walker AP, Balls M. A prevalidation study on in vitro skin corrosivity testing. ATLA 23:219-255 (1995)
Boukamp P, Dzarliewa-Petrusevska RT, Breitkreutz D, Hornung J, Markham A, Fusenig NE. Normal keratinization in a spontaneously immortalized aneuploid human keratinocyte line. J. Cell. Biol. 106:761-771 (1988)
Boukamp P, Popp S, Bleuel K, Tomakidi E, Bürkle A, Fusenig,NE. Tumorigenic conversion of immortal human skin keratinocytes (HaCaT) by elevated temperature. Oncogene 18: 5638-5645 (1999)
Breitkreutz D, Schoop VM, Mirancea N, Baur M, Stark H-J, Fusenig NE. Epidermal differentiation and basement membrane formation by HaCaT cells in surface transplants. Eur. J. Cell Biol. 75:273-286 (1998)
Buzin CH, Bournias-Vardiabasis N. Teratogens induce a subset of small heat shock proteins in Drosophila primary embryonic cell cultures. Proc. Natl. Acad. Sci. USA 81:4075-4079 (1984)
Damour O, Augustin C, Black AF. Cellular engineering: bioengineering of skin. Applications of reconstructed skin models in pharmaco-toxicological trials. Med. Biol. Eng. Comput. 36:825-832 (1998)
Draize JH, Woodward G, Calvery OH. Methods for the study of irritation and toxicity of substances applied topically to the skin and mucous membranes. J. Pharmacol. Exp. Therap. 82:377-390 (1944)
Dreano et al., Gene 49: 1-8 (1987)
Dreano et al., Virus Research 8: 43-59 (1987)
Dreano et al., Biotechnology 6: 953-958 (1988)
Fentem JH, Archer GEB, Balls M, Botharn PA, Curren RD, Earl LK, Esdaile DJ. The ECVAM international validation study on in vitro tests for skin corrosivity. 2. Results and evaluation by the management team. Toxic. In Vitro 12:483-524 (1998)
Hickay, E., Brendon, S.E., Potter, R., Stein, G. und L.E. Weber, Nucleic Acid Res. 14: 4127-4144 (1986)
Kretz, C., Melen, P., Mireault, M.E., Arrigo, A. P, J. Cell Biol. 133: 1083-1093 (1996)
Kretz, C. and Arrigo, A. P., FEBS Lett. 351: 191-196 (1994)
Nickoloff, B.J., Naidu, Y., Clinical and laboratory studies. Perturbation of epidermal barrier function correlates with initiation of cytokine cascade in human skin, J. Am. Acad. Dermatol. 30: 535-546 (1994)
Rinderle, C.; Dissertation: Etablierung und Charakterisierung zweier zellulärer Systeme zur Unterscheidung zwischen p53 Wildtyp und p53 Mutantenfunktion, Freiburg 1998
Saliou C, Kitazawa M, McLaughlin L, Yang J-P, Lodge JK, Tetsuka T, lwasaki K, Cillard J, Okamoto T, Packer L. Antioxidants modulate acute solar ultraviolet radiation-induced NFkappa-B activation in a human keratinocyte cell line. Free Radic. Biol. Med. 26:174-183 (1999)
Schoop VM, Mirancea N, Fusenig NE. Epidermal organization and differentiation of HaCaT keratinocytes in organotypic coculture with human dermal fibroblasts. J. Invest. Dermatol. 112:343-353 (1999)
Spielmann H, Balls M, Dupuis J, Pape WJ, Pechovitch G, de Silva O, Holzhütter H-G, Clothier R, Desolle P, Gerberick F, Liebsch M, Lovell WW, Maurer T, Pfannenbecker U, Potthast JM, Csato M, Sladowski D, Steiling W, Brantom P. The international EU/COLIPA in vitro phototoxicity validation study: results of phase II (blind trial). Part 1: the 3T3 NRU phototoxicty test. Toxic. in Vitro 12.305-327 (1998)
Tupker RA, Willis C, Berardesea E, Lee CH, Fartasch M, Agner T, Serup J. Guidelines on sodium lauryl sulfate (SDS) exposure tests. A report from the Standardization Group of the European Society of Contact Dermatitis. Contact Dermatitis 37:53-69 (1997)
Van de Sandt J, Roguet R, Cohen C, Esdaile D, Ponee M, Corsini E, Barker C, Fusenig NE, Liebsch M, Benford D, Brugerolle de Fraissinette A, Fartasch M. The use of human keratinocytes and human skin models for predicting skin irritation. ATLA 27: 723-743 (1999)
van Ruissen F, Le M, Carroll JM, van der Valk PGM, Schalkwijk J. Differential effects of detergents on keratinocyte gene expression. J. Invest. Dermatol. 110:358-363 (1998)
Weber C, Podda M, Rallis M, Traber MG, Packer L. Efficacy of topical application of tocopherols and tocotrienols in protection of murine skin from oxidative damage induced by UV-irridiation. Free Radie. Biol-. Med. 761-769 (1997)

## Claims

1. An immortalized human skin keratinocyte line stably transfected with a vector, said vector comprising
(a) a promoter inducible by environmental hazards; and
(b) a reporter gene in operative linkage thereto.

2. The immortalized human skin keratinocyte line of claim 1, which is HaCaT.

3. The immortalized human skin keratinocyte line of claim 1 or 2, wherein the environmental hazard is heat, ultraviolet irradiation (UVR), ozone or an organic or anorganic compound.

4. The immortalized human skin keratinocyte line of claim 3, wherein the organic or anorganic compound is a metal, pesticide, herbicide, biocide, industrial residue or a combustion product.

5. The immortalized human skin keratinocyte line of any one of claims 1 to 4, wherein the promoter is which is the HSP-70- or HSP-27-promoter or an NF_{κ}B- or AP-1-site containing promoter.

6. The immortalized human skin keratinocyte line of any one of claims 1 to 5, wherein the reporter gene is the gene encoding luciferase, chloramphenicol acetyl transferase (CAT), green fluorescent protein (GFP) or human placental secreted alkaline phosphatase (SEAP).

7. An in vitro toxicity assay, comprising
(a) culturing the immortalized human skin keratinocyte line of any one of claims 1 to 6 in the presence of the candidate environmental hazard under conditions such that the protein encoded by the reporter gene is expressed depending on induction of the promoter; and
(b) determining whether expression of the reproter gene occures,
wherein expression of the reporter gene is indicative of cell toxicity of the candidate environmental hazard.

8. An in vitro toxicity assay, comprising
(a) culturing the immortalized human skin keratinocyte line of any one of claims 1 to 6 in (i) the presence and (ii) absence of the candidate environmental hazard under conditions such that the protein encoded by the reporter gene is expressed depending on induction of the promoter; and
(b) comparing the expression rate of the reproter gene of (i) and (ii)
wherein an increase of the expression rate of (i) compared to (ii) is indicative of cell toxicity of the candidate environmental hazard.
